# EUROPEAN PATENT APPLICATION

(11) **EP 0 657 542 A1**
(43) Date of publication of application: **14.06.1995**
(21) Application number: 94309047.2
(22) Date of filing: 06.12.1994
(51) Int. Cl.: C12P 7/56

(54) **Method for fermentative production of lactic acid**

(30) Priority: 08.12.1993 JP 308347/93
(71) Applicant: MUSASHINO CHEMICAL LABORATORY Ltd., Tokyo (JP)
(72) Inventor: Miura, Shigenobu, Suginami-ku, Tokyo (JP); Kumagai, Akira, Hidaka-shi, Saitama-ken (JP)
(74) Representative: Rees, David Christopher

(57) **Abstract**

A method for producing a lactate, particularly ammonium lactate, by electrodialytic fermentation. The production is accomplished by a method of fermentation which comprises circulating a fermentation liquor between a fermentation part for culturing a microorganism capable of accumulating a lactate in a culture medium and an electrodialytic apparatus, and removing the formed lactate from the fermentation part by means of the electrodialytic apparatus.

## Description

This invention relates to a novel method for the fermentative production of lactic acid.

The lactic acid is utilized as a food additive for the production of sake (an alcoholic beverage made in Japan by the fermentation of rice), refreshing beverages, pickles, soysauce, bread, or beer and is also utilized industrially for the production of leather, textiles, plastics, medicines, or agricultural chemicals.

Recently, the derivatives or the intermediates for synthesis of lactic acid, namely the lactic esters such as ethyl lactate and butyl lactate, have been finding growing utility as solvents and detergents of high safety. Further, polylactic acid which is a polymer of lactic acid is expected to find growing utility as a biodegradable polymer.

Heretofore, as means to produce lactic acid, a fermentative method using sugars and starch as raw materials and resorting to the action of lactic acid bacteria has been widely adopted. This method generally implements the production of lactic acid by using whey, corn steep liquor, yeast extract, etc. as nutrient sources for lactic acid bacteria in a culture medium, adding to the culture medium calcium carbonate as a neutralizing agent for lactic acid to be formed therein, and inoculating lactic acid bacteria to the culture medium thereby inducing fermentative formation of lactic acid.

The method which relies on calcium carbonate to neutralize the lactic acid being accumulated in the culture medium, however, not merely consumes much time and labor in attaining necessary purification but also yields the fermentation to inhibition by the formed lactic acid. Particularly when the lactic acid formed in the culture medium accumulates to a high concentration, the rate of fermentation is retarded and the productivity of the apparatus of fermentation is seriously impaired.

As means to heighten the productivity of the apparatus for fermentation, methods of high-density culture which adopt such measures as immobilization of bacterial cells or separation of bacterial cells by a membrane have been developed. These methods of fermentation, however, are not immune from the inhibition of the fermentation by the produced lactic acid.

As means to eliminate the inhibition of fermentation by the product and heighten the productivity by separating from the culture medium for fermentation the produced lactic acid, extract fermentation and electrodialytic fermentation are conceivable.

The extract fermentation is a method which comprises continually extracting for recovery the lactic acid accumulating in the culture medium thereby eliminating the inhibition by the product and, at the same time, attaining crude formation of the product. This method, however, has not been fully developed so far because the optimum pH for fermentation and the optimum pH for extraction are different and also because an extractant used for the extraction generally exerts on bacterial cells such toxicity as gains in intensity in proportion as the efficacy thereof exalts.

The electrodialytic fermentation was first developed by Nomura et al. (Applied and Environmental Microbiology, Aug. 1986, pp. 314 to 319). The apparatus for this electrodialytic fermentation is constructed by having a cathode, an anion-exchange membrane, a cation-exchange membrane, and an anode sequentially arranged in the order mentioned and is operated by introducing a fermentation liquor into a cathode compartment and dialyzing the produced lactic acid through the anion-exchange membrane. Thus, this method is capable of obtaining free lactic acid.

This method, however, permits no ample increase of the productivity because the pH of the culture medium for fermentation is controlled by the electrodialysis of the lactic acid and, as a result, the lactic acid concentration in the fermentation liquor is lowered and the current efficiency of the electrodialysis is proportionately lowered and further because phosphoric acid ions are dialyzed together with lactic acid and the bacterial cells in the cathode compartment are destroyed on contact with the electrodes and the anion-exchange membrane.

Toda et al. have demonstrated that the electrodialytic fermentation is effected more efficiently by causing the lactic acid in the fermentation liquor to exist in the form of a salt instead of a free lactic acid thereby alleviating the inhibition by the product and consequently enabling the fermentation to proceed amply and quickly even when the lactic acid concentration in the fermentation liquor is adjusted in the range of from 2 to 3%, preventing the bacterial cells from destruction by dint of the separation of bacterial cells with a membrane, leading the fermentation liquor into a cathode compartment of an electrodialytic apparatus constructed by having a cathode, an anion-exchange membrane, a cation-exchange membrane, and an anode sequentially arranged in the order mentioned, and inducing dialysis of the lactic acid therein (J. Gen. Appl. Microbiol., 36, 111-125, 1990).

Boyaval et al. also have demonstrated that dialysis of sodium lactate is attained by leading the fermentation liquor through an ultrafilter membrane into an electrodialytic apparatus having a plurality of sets of a cation-exchange membrane and an anion-exchange membrane interposed between a cathode and an anode (Biotechnology Letters, Vol. 9, No. 3, pp. 207 to 212, 1987).

A method which is capable of recovering lactic acid of high purity economically and efficiently from sodium lactate and permitting reclamation of sodium has not been known to the art to date.

When the bacterial cells are subjected to precision filtration or ultrafiltration before the fermentation liquor is led to the electrodialytic apparatus as proposed by Toda et al. or Boyaval et al., since the fermentation liquor subjected to filtration is required to be in such a state as contains the lactic acid at a low concentration for the purpose of exalting the productivity while keeping the lactic acid concentration in the fermentation liquor at a low level, the amount of the liquor to be passed for the filtration in this case is several times to some tens of times the amount of the fermentation liquor containing the lactic acid at a concentration in the neighborhood of 10% as in the ordinary continuous fermentation and the surface area of the membrane is proportionately large. Thus, the methods under discussion are at a disadvantage in being uneconomical.

It has been known that ammonium lactate is easily esterified by an alcohol having four or more carbon atoms. A method for obtaining lactic acid of high purity efficiently from a fermentation liquor producing ammonium lactate therein has been already disclosed by Miura, one of the present inventors, and his associates (Japanese Patent Application 05-230,428). This method comprises adding n-butanol to a fermentation liquor containing ammonium lactate thereby promoting an esterification and, at the same time, effecting liberation for recovery of the greater part of ammonia, then adding sulfuric acid to the fermentation liquor thereby further promoting the esterification and consequently inducing efficient formation of butyl lactate, and subsequently purifying the butyl lactate by distillation and hydrolyzing the purified butyl lactate thereby obtaining lactic acid of high purity. A method which economically produces ammonium lactate by the use of a practical apparatus for fermentation while precluding the inhibition by a product has not been known to the art to date.

In view of the various problems mentioned above, the present invention has for an object thereof the provision of a novel method for the fermentation of lactic acid.

Another object of this invention is to provide a method for obtaining lactates, particularly ammonium lactate, by practicable electrodialytic fermentation.

The present inventors, resolved to solve the problems mentioned above, made a diligent study in search for a novel method for the fermentative production of lactic acid. As a result, it has been ascertained that the productivity of a fermentation apparatus can be heightened by adjusting the pH of fermentation by the addition of an alkali and leading a fermentation medium containing bacterial cells to a dilution chamber of an electrodialytic apparatus and dialyzing a lactate therein thereby attaining easy removal of the lactate from the fermentation liquor at a high current efficiency. This invention has been perfected as a result.

Specifically, the objects of this invention are accomplished by (1) a method for the fermentative production of lactic acid characterized by circulating a fermentation liquor between a fermentation part for culturing a microorganism capable of accumulating a lactate in a culture medium and an electrodialytic apparatus and removing the lactate from the fermentation liquor by the electrodialytic apparatus.

The objects of this invention are further accomplished by (2) the method indicated in (1) above, wherein the operation of the electrodialytic apparatus is carried out intermittently.

The objects of this invention are further accomplished by (3) the method indicated in (1) above, wherein the electrodialytic apparatus is connected to a plurality of fermentation parts and fermentation liquors in the plurality of fermentation parts are alternately circulated between the electrodialytic apparatus and the fermentation parts.

The objects of this invention are further accomplished by (4) the method indicated in (2) or (3) above, wherein the raw materials for fermentation are supplied by way of replenishment to the fermentation parts while the operation of the electrodialytic apparatus is suspended.

The objects of this invention are further accomplished by (5) the method indicated in (4) above, wherein the raw materials for fermentation supplied by way of partial replenishment to the fermentation parts are sugars and/or inorganic salts.

The objects of this invention are further accomplished by (6) the method indicated in (1) above, wherein the operation of the electrodialytic apparatus is controlled by the degree of electroconductivity of the culture medium for fermentation.

The objects of this invention are further accomplished by (7) the method indicated in (1) above, wherein the microorganism capable of accumulating a lactate is a microorganism belonging to one of the genera Lactobacillus, Lactococcus, Bacillus, and Sporolactobacillus.

The objects of this invention are further accomplished by (8) the method indicated in (7) above, wherein a fermentation liquor from which the cells of the microorganism have been separated through the medium of a microorganic cell separation device is led to the electrodialytic apparatus.

The objects of this invention are further accomplished by (9) the method indicated in (1) above, wherein the microorganism capable of accumulating a lactate is a microorganism belonging to genus Rhizopus and a fermentation liquor having microorganic cells therein sterilized through the medium of a sterilizing device is led to the electrodialytic apparatus.

The objects of this invention are further accomplished by (10) the method indicated in (1) above, wherein the culture medium and the microorganic cells in the fermentation part are partly extracted and a fresh supply of culture medium is added to the fermentation part to allow the fermentation to proceed continuously.

The objects of this invention are further accomplished by (11) the method indicated in (1) above, wherein the lactate is ammonium lactate and the p-H of the culture medium for fermentation is controlled by ammonia.

The methods of this invention are at a great advantage in enabling the aqueous solution of a crudely refined lactate, particularly ammonium lactate, to be obtained easily and, at the same time, exalting the productivity of a fermentation apparatus.

In the drawings:-
Fig. 1 illustrates one embodiment of the operation of this invention,
Fig. 2 illustrates one embodiment of an electrodialytic apparatus according to this invention,
Fig. 3 is a schematic diagram illustrating an experimental apparatus involved in Examples 1 to 3 of this invention, and
Fig. 4 is a schematic diagram illustrating an experimental apparatus involved in Example 4.

Now, this invention will be described in detail below.

Heretofore, the electrodialytic fermentation for the production of lactic acid has been developed mainly with respect to systems intended to give free lactic acid as the product. The methods of these systems come under two types, those of the one type using a procedure which comprises electrodialyzing free lactic acid in a fermentation liquor and those of the other type using a procedure which comprises provisionally converting lactic acid into a salt and dialytically separating lactic acid from a fermentation liquor by dint of electrolysis. As a fault common to these methods, the fact that since they require the fermentation liquor to be passed to the cathode compartment, the microorganic cells are killed on the surfaces of a cathode and an anion-exchange membrane may be cited. To repress this fault, it is customary to adopt a method which comprises passing the fermentation liquor through a separation device using a membrane or a centrifugal separation device to separate microorganic cells from the fermentation liquor in advance of the step of leading the fermentation liquor to the electrodialytic apparatus and then returning the separated microorganic cells to the fermentation part. Since the lactic acid concentration in the fermentation part is required by the essential nature of the electrodialytic fermentation to be kept at a low level for the purpose of alleviating the inhibition by the product, the lactic acid concentration in the microorganic cell separating part is low and the amount of the fermentation liquor to be treated is inevitably large relative to the amount of lactic acid to be separated by electrodialysis. This fact constitutes a serious problem. As one way of preventing the microorganic cells from contacting the cathode, an idea of interposing another anion-exchange membrane between the cathode and the anion-exchange membrane and passing the fermentation liquor between the two opposed anion-exchange membranes may be conceived. Since the electrodialytic cell is caused by electrolysis to produce a pH gradient from the inlet to the outlet thereof, the destruction of microorganic cells occurs nevertheless and the increase of number of anion-exchange membranes gives rise to electric resistance, with the result that the working voltage will rise and the electric power consumption will increase.

Further, since the electrodialysis effects separation of lactic acid or a lactate from the other components of the fermentation liquor, the product is obtained in a more or less crudely refined state. The product of this purity, however, is not fully suitable for use as a food additive or in industrial production. For the product of this separation to acquire high purity, it is required to undergo an additional refining process. As a potential way of obtaining lactic acid of high purity, the aforementioned method for directly esterifying ammonium lactate with n-butanol is available. This method fulfills its function amply by simply obtaining ammonium lactate and is not required at all to obtain lactic acid in a free form in the fermentation part. The method which esterifies ammonium lactate with n-butanol allows the fermentation liquor containing ammonium lactate to be put to use in its unmodified form, namely without requiring separation of the microorganic cells, for the next process. Incidentally, the adhesion of the components of the culture medium for fermentation to the reaction part possibly occurs heavily, depending on the raw materials for fermentation. Thus, the fermentation liquor containing ammonium lactate is desired to have undergone crude refinement. Even where the fermentation liquor containing ammonium lactate can be used in its unmodified form, the crude refinement which has been given thereto allows the amount of the residue of the fermentation liquor remaining after the separation of butyl lactate by distillation to decrease and also permits the viscosity of the residue to decrease, with the result that the refinement by distillation will proceed smoothly and the improvement of the yield of the distillation will be accomplished.

It will be easily understood that in this circumstance, the method of the present invention is at an advantage in obtaining easily an aqueous solution of a crudely refined lactate, particularly ammonium lactate and, at the same time, exalting the productivity of the fermentation apparatus.

Now, the method of this invention for the fermentative production of lactic acid will be described more specifically below.

First, the microorganism which can be used for the present invention is not particularly limited but is only required to be capable of accumulate a lactate. As concrete examples of the microorganism, lactic acid bacteria of genus Lactobacillus, genus Lactococcus, genus Bacillus, and genus Sporolactobacillus and fung; of genus Rhizopus may be cited. The term "microorganism" as used herein means yeasts, molds, fungi, bacteria, ray fungi, unicellular algae, viruses, and protozoans and further embraces cells not differentiated between animals and plants and tissue cultures.

The lactate to be accumulated by the microorganism is desired to be ammonium lactate on account of the convenience with which lactic acid of high purity will be obtained by the next process of refinement. It does not need to be limited to ammonium lactate but may be sodium lactate or potassium lactate instead. These salts are soluble in water and perfectly innocuous and, therefore, rated as GRAS by FDA and allowed to be used as a food additive in the place lactic acid.

Then, the culture medium to be effectively used in this invention may be any of the conventional culture media which are used for culturing microorganisms. As concrete examples of the culture medium usable herein, YM culture medium, potato-glucose culture medium, Gorodkova culture medium, sodium acetate culture medium, wort culture medium, malt extract culture medium, vegetable juice culture medium, gypsum culture medium, cornmeal culture medium, potato yeast steep liquor, glucose culture medium, Czapek's culture medium, Sabouraud's culture medium, oatmeal culture medium, synthetic Mucor culture medium, YpSs culture medium, glucose-dry yeast culture medium, bouillon culture medium, yeast-malt culture medium, starch-inorganic salt culture medium, glycerin-asparagine culture medium, peptone-yeast-iron culture medium, thyrosine culture medium, and Pridham-Gotrieb culture medium may be cited. These culture media may be used in a liquid form or in a form solidified as with gelatine or agar.

Though the raw materials for fermentation which can be used for this invention are variable with the kind of a microorganism to be used, they are basically required only to be assimilable. The raw materials which are generally used for the fermentative production of lactic acid include D-glucose, sucrose, lactose, D- and L-arabinose, D-ribose, D-xylose, D-mannose, D-galactose, L-rhamnose, D-fructose, L-sorbose, maltose, lactose, melibiose, cellobiose, trehalose, raffinose, melezitose, α methyl-D-glucoside, D-glucosamine, N-acetylglucosamine, arbutin, dextrin, soluble starch, inulin, methanol, ethanol, adonitol, erythritol, inositol, D-mannitol, D-sorbitol, dulcitol, D-gluconate, glycerin, hexadecane, and starch, for example. Sugar sources which contain such impurities as blackstrap molasses and whey are also usable.

When the microorganism to be used is lactic acid bacteria of genus Lactobacillus, genus Lactococcus, genus Bacillus, or genus Sporolactobacillus, it is customary to add as secondary raw materials such nitrogen sources as yeast extract, peptone, and corn steep liquor which are essential for the growth of the microorganism. It is also permissible to add such inorganic salts as potassium primary phosphate, potassium secondary phosphate, magnesium sulfate, sodium chloride, ferrous sulfate, manganese sulfate, manganese chloride, zinc sulfate, and calcium carbonate when necessary. In the case of the microorganism of genus Rhizopus, it suffices to add such inorganic salts as mentioned above besides sugars and other raw materials.

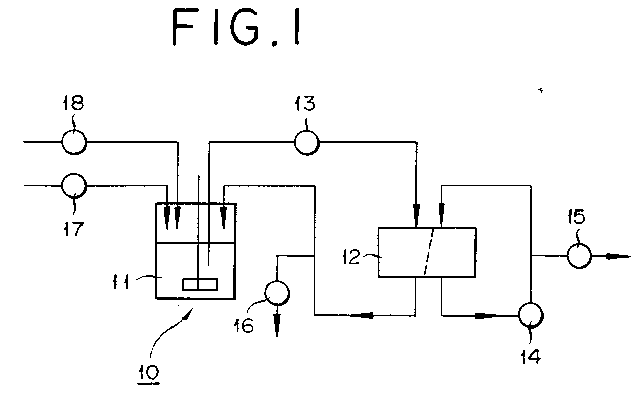

Now, a fermentation apparatus for the production of lactic acid in accordance with the method of this invention will be described below with reference to Fig. 1 which schematically illustrates the fermentation apparatus.

With reference to Fig. 1, in a fermentation apparatus 10 for the production of lactic acid, when a fermentation liquor is circulated by means of a fermentation liquor circulation pump 13 between a fermentation part 11 for culturing a microorganism capable of accumulating a lactate in a culture medium and an electrodialytic apparatus 12 and is consequently dialyzed by means of the electrodialytic apparatus 12, a concentrated lactate is extracted from the fermentation liquor and recovered via a recovered lactate circulation pump 14 by means of a lactic acid extraction pump 15 and the fermentation liquor from which the lactate has been extracted is returned meanwhile to the fermentation part 11. Part of the fermentation liquor so returned is extracted out of the fermentation apparatus 10 by means of a waste microorganic cell-waste culture medium extraction pump 16 for the purpose of preventing the amount of dead microorganic cells and waste matter of fermentation suffered to accumulate in the culture medium from increasing excessively. Further in the fermentation apparatus 10 for the production of lactic acid, the raw materials for fermentation are suitably fed by a raw material pump 17 to the fermentation part 11 so as to ensure production of the lactate by continuous fermentation and the pH of the culture medium for fermentation in the fermentation part 11 is measured and, when the pH so measured requires an adjustment to the prescribed level which will be specifically described hereinbelow, the aqueous solution of an alkali is fed to the culture medium by means of an alkali supply pump 18.

Of the component parts of the fermentation apparatus 10 mentioned above, the fermentation part 11 is not particularly limited. It may be an ordinary stirring type fermentation tank, a column type reactor packed with gel beads such as of calcium alginate having propagating microorganic cells immobilized thereon, or a membrane type reactor having microorganic cells occluded at a high density in a membrane. The fermentation part 11 is desired to be provided with various sensors and thereby enabled to determine such magnitudes as the lactate concentration and the pH or electroconductivity of the culture medium for fermentation as will be specifically described hereinbelow.

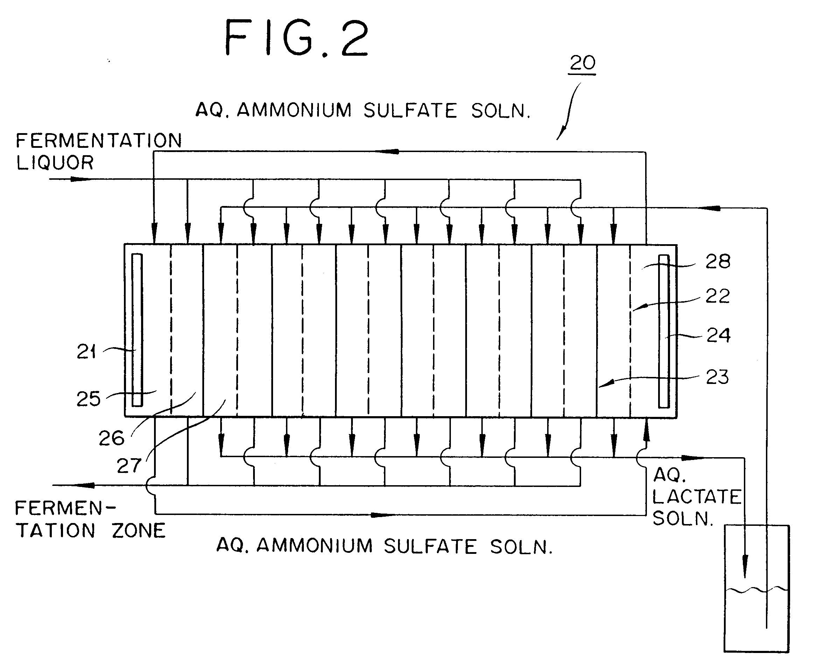

The electrodialytic apparatus 12 is only required to be capable of dialyzing the lactate from the fermentation liquor and is not restricted on account of the type of construction. For example, an electrodialytic apparatus which is adapted to operate continuously and is constructed so as to incorporate a plurality of dialytic cells therein as illustrated in Fig. 2, a diagram schematically depicting the construction of an electrodialytic apparatus to be used for working the method of this invention, may be used.

As illustrated in Fig. 2, the electrodialytic apparatus 20 to be used for the method of this invention, in a general construction, has a cathode 21, a plurality (seven as shown in the diagram) of sets of a cation-exchange membrane 22 and an anion-exchange membrane 23, a cation-exchange membrane 23, and an anode 24 sequentially arranged in the order mentioned so that the parts partitioned by the ion-exchange membranes 22 and 23 may sequentially form a cathode compartment 25, a plurality (seven as shown in the diagram) of pairs of a dilution chamber 26 and a concentration chambers 27, and an anode compartment 28. When the fermentation liquor is passed to the dilution chambers 26, alkali ions move in the direction of the cathode 21 through the cation-exchange membranes 22 and the lactate ions move in the direction of the anode 24 through the anion-exchange membranes 23 and the lactate is concentrated in the concentration chambers 27. When the pH adjustment is effected with ammonia, ammonium sulfate is circulated to the cathode compartment 25 and the anode compartment 28. As a result, the liquids in the cathode compartment 25 and the anode compartment 28 are alternately circulated and the alkali which has moved into the cathode compartment 25 from the dilution chamber 26 adjoining the cathode compartment 25 moves from the anode compartment 28 to the concentration compartment 27 adjoining the anode compartment 28. In the electrodialytic apparatus 20, since the fermentation liquor is not allowed either to contact the electrodes 21 and 24 or to form any noticeable pH gradient inside the dilution compartments 26, the microorganic cells contained in the fermentation liquor are not killed even when the fermentation liquor is directly fed to the electrodialytic apparatus 20. This fact constitutes one of the characteristic features of the present invention.

Further, the electrodialytic apparatus 20 effects crude refinement of the lactate to a full extent because it removes not only the microorganic cells but also nonionic organic substances and macromolecular compounds from the lactate obtained by the electrodialysis. It also permits the separation of the microorganic cells to be attained with a greater saving of power than the ordinary separation by the passage of the fermentation liquor through membranes because the amount of dialysis in the electrodialytic apparatus 20 is small and the amount of microorganic cells suffered to deposit on the surfaces of the ion-exchange membranes 22 and 23 is proportionately small.

In the case that the continuous fermentation brings about an increase in the density of microorganic cells and, as a result, the ion-exchange membranes 22 and 23 of the electrodialytic apparatus 20 are clogged or the current efficiency is seriously degraded, however, it is necessary that the microorganic cells be separated and removed from the fermentation liquor before the fermentation liquor is passed to the electrodialytic apparatus 20. The device to be used for this separation is not particularly limited. It may be any of the conventional devices which are adapted to effect the separation by the use of membranes or by virtue of centrifugal separation.

When the device for separating microorganic cells is installed separately of the electrodialytic apparatus, the fact that this device is inevitably voluminous as mentioned above turns out to be an economic handicap. The electrodialytic apparatus to be used for this invention is far less expensive on account of the construction thereof than the electrolytic apparatus used for the production of free lactic acid. Thus, the entire system inclusive of the device mentioned above proves to be sufficiently economic.

When the microorganic cells are those of a microorganism of genus Rhizopus, it is no longer necessary to install the special device for separation mentioned above because the separation of the culture medium and the microorganic cells is easily attained. It is, however, necessary in this case to install a sterilizing device capable of destroying the microorganic cells for the purpose of preventing part of hyphae or spores from flowing into the electrodialytic apparatus and the microorganic cells from being immobilized on the electrodialytic apparatus and being grown therein. The sterilizing device in this case is desired to be of a type resorting to the action of heat on account of the simplicity of operation. It is naturally allowable to adopt a device. using other means of sterilization instead.

Then, in the method of this invention for electrodialytic fermentation, the pH of the culture medium for fermentation is desired to be kept controlled throughout the entire process of fermentation in the optimum range of from 4 to 8, preferably from 5 to 7, by the addition of an alkali for the sake of the culture of the microorganism in the fermentation part and particularly by the addition of ammonia or aqua ammonia for the sake of the subsequent process of refinement. In the method of this kind, the fermentation is enabled to proceed highly efficiently without entailing any inhibition by the product by separating the formed lactate from the fermentation liquor by means of electrodialysis, recovering the dialyzed lactate, causing the fermentation liquor remaining after the dialysis to be returned through a circulation system to the fermentation part, and controlling the pH value, the electroconductivity, and the lactate concentration of the whole culture medium for fermentation including the fermentation liquor at desired levels.

The rate of the fermentation can be exalted by keeping the lactate concentration in the fermentation liquor existent in the fermentation part at the lowest possible level. When the lactate concentration is lowered, however, the electroconductivity of the culture medium for fermentation is proportionately lowered, the current efficiency of the electrodialysis handling the fermentation liquor of this quality is degraded, the productivity of the electrodialytic apparatus is impaired and, at the same time, the amount of other components to be dialyzed is increased, and the selectivity of the fermentation in process is consequently impaired. The lactate concentration in the fermentation liquor, therefore, is desired to be in the range of from 0.2 to 2% by weight.

The control of the lactate concentration in the fermentation liquor can be attained by any of the conventional methods. When the lactate concentration of the fermentation liquor is low, the rate of fermentation can be exalted, however, the productivity of the electrodialytic apparatus is degraded it the electrodialytic apparatus is operated at such low lactate concentration of the fermentation liquor continuously, because the electric current per unit surface area of the ion-exchange membranes is lowered and the current density is consequently lowered. The system of high operational efficiency can be attained by setting the upper and the lower limit of the lactate concentration in the fermentation liquor and repeating the start and the stop of the operation of the electrodialytic apparatus through the ON-OFF control of the lactate concentration within the range. In the case that the fermentation liquor having the microorganic cells not separated therefrom is directly circulated to the electrodialytic apparatus, the electrodialytic apparatus so constructed as illustrated in Fig. 2, for example, is desired to be designed so that the operation of dialysis of the lactate alone may be stopped by suspending the flow of electric current to the electrodes of the electrodialytic apparatus instead of stopping the circulation of the fermentation liquor for preventing the microorganic cells from being deposited inside the electrodialytic apparatus and the fermentation liquor may be passed and circulated downwardly through the dilution chambers partitioned by the ion-exchange membranes.

The suspension of the flow of electric current to the electrodes of the electrodialytic apparatus directly results in a decrease of the rate of operation of the electrodialytic apparatus. The rate of operation of the electrodialytic apparatus can be exalted virtually to 100% by causing desirably one electrodialytic apparatus to take charge of a plurality of fermentation parts and switching these fermentation parts with respect to their selective engagement in the circulation of the fermentation liquor to the electrodialytic apparatus so as to control the lactate concentration.

The time for starting or stopping the operation of the electrodialytic apparatus or the time for switching the fermentation parts mentioned above can be easily selected by measuring the electroconductivity of the culture medium for fermentation liquor in the fermentation part. It is conventional to control the electrodialysis by the electroconductivity. In the conventional method for electrodialytic production of lactic acid, the measurement of the lactate concentration in the fermentation liquor in the fermentation part has been relied on for the control of the electrodialytic apparatus. It has been ascertained by the present inventor's study that even in the culture medium for fermentation which contains other inorganic salts and extraneous substances copiously and possesses a large buffer capacity, the lactate concentration can be estimated satisfactorily by the measurement of the electroconductivity. By this knowledge, the method of this invention is enabled to effect the control of the electrodialytic fermentation by the use of an electroconductivity sensor very simple as compared with the like sensor used in the conventional method of electrodialytic fermentation for the production of lactic acid. Since the electroconductivity under consideration is correlated with the lactate concentration, it is only required to be such as to correspond to the lactate concentration falling in the range of from 0.2 to 2% by weight mentioned above. Specifically, this electroconductivity is desired to be adjusted so as to fall in the range of from 1 to 30 mS/cm, preferably from 5 to 15 mS/cm. When the electrodialytic apparatus is intermittently operated as described above, the upper and the lower limit are set on the electroconductivity of the fermentation part so that the operation of the electrodialytic apparatus will be started when the concentration of the lactate rises in consequence of the advance of the fermentation in the fermentation part and the electroconductivity of the culture medium for fermentation reaches the upper limit and the operation of the electrodialytic apparatus will be stopped when the lactate concentration in the fermentation part is lowered by the advance of electrodialysis and the electroconductivity of the culture medium for fermentation reaches the lower limit. When a plurality of fermentation parts are assigned to one electrodialytic apparatus, the control of the electrodialytic fermentation is attained by setting the lower limit on the electroconductivity and repeating a procedure which comprises switching the fermentation part currently engaging in dialysis, when the electroconductivity of the culture medium for fermentation in that fermentation part reaches the lower limit, to another fermentation part in which the electroconductivity of the culture medium for fermentation is higher than the lower limit thereby continuing the operation successively with the plurality of fermentation parts.

Concerning the control of the electrodialytic fermentation, the method of fermentation which necessitates electrodialysis of the lactate is at an advantage in enabling the system to operate stably without entailing any noticeable trouble because the pH of the culture medium for fermentation in the fermentation part is controlled by the addition of an alkali such as aqua ammonia separately of the control of the electrodialysis and, therefore, an accidental increase of the lactate concentration in the fermentation part while in operation brings about virtually no trouble.

As respects the selectivity of the electrodialysis, it is apparent that the inorganic salts which are other essential components for the culture of the microorganism are dialytically removed from the fermentation liquor at the time that the lactate is dialyzed. In the case of the electrodialytic fermentation which is performed batchwise, however, it has been ascertained that when the inorganic salts are made to exist in a suitable amount in the culture medium before the fermentation is started, the fermentation can be completed without requiring the fermentation liquor to be replenished with inorganic salts while the fermentation is in progress. This situation may be explained by a supposition that the inorganic salts existing at first are contained within the microorganic cells during the initial stage of fermentation and the microorganic cells afford an ample supply of inorganic salts for one batch of fermentation. The corn steep liquor which sufficiently contains essential inorganic salts, for example, does not need to be replenished with inorganic salts no matter whether the electrodialytic fermentation is performed batchwise or continuously. In the case that the yeast extract is used as a nutrient source and the separate addition of inorganic salts is necessary, the microorganism is enabled to enjoy efficient use of inorganic salts by causing the inorganic salts to exist in a small amount at the start of the fermentation for the purpose of exalting the selectivity of electrodialysis and preventing loss of inorganic salts and, after the start of the electrodialysis, feeding inorganic salts for the purpose of replenishment to the fermentation part when the operation of the electrodialytic apparatus is suspended in the method of control of the electrodialysis mentioned above or when the fermentation part previously engaging in the electrodialysis is switched to another fermentation part and is consequently disjoined from the electrodialytic apparatus.

The sugars which form main raw materials for fermentation to be contained in the culture medium in the fermentation part are D-glucose, sucrose, and lactose, for example, as mentioned above. Since these sugars are moved from the dilution compartments to the concentration compartments through the intervening ion-exchange membranes in the apparatus constructed as illustrated in Fig. 2 mentioned above at the same time that the lactate is electrodialyzed, the ratio of circulation and recovery of sugar contents is decreased and the selectivity thereof is proportionately degraded. Generally, the ease with which a given substance is passed through an ion-exchange membrane increases in proportion as the molecular weight of the substance decreases. In the case that glucose is used as a sugar component of the raw materials for the batchwise electrodialytic fermentation, for example, when the sugar concentration in the charged raw material is 10% by weight, 5 to 10% by weight of the charged glucose is inevitably moved toward the concentration compartments at the end of fermentation, though variable with the kind of ion-exchange membrane and the operating conditions of electrodialysis. The loss of the sugar component due to the passage thereof to the concentration parts, therefore, can be repressed to a low level by adding the sugar component in such an amount as to keep the sugar concentration in the fermentation liquor may be kept constantly at a low level similarly to the inorganic salts and, particularly after the start of the operation of electrodialysis, feeding the sugar component for the purpose of replenishment to the fermentation part when the operation of the electrodialytic apparatus is suspended in the method of control of the electrodialysis mentioned above or when the fermentation part previously engaging in the electrodialysis is switched to another fermentation part and is consequently disjoined from the electrodialytic apparatus.

Otherwise, the loss of the raw material for fermentation can be generously repressed by using starch, a substance having a large molecular weight, or dextrin, a partial hydrolyzate of starch, as the raw material for fermentation. Often, such substances as starch which generally have large molecular weights are not easily assimilated by lactic acid bacteria. In this case, the fermentation liquor may incorporate a liquefied enzyme or a saccharified enzyme as a supplementary component.

When the electrodialytic fermentation of this invention is adapted to be continuously operated, the process of high-density culture may be adopted for the microorganic cells in the culture medium for fermentation. The continuous electrodialytic fermentation is enabled to acquire higher productivity than the batchwise electrodialytic fermentation by continuously or intermittently feeding a fresh supply of culture medium to the fermentation part and removing and recovering the formed lactate by electrodialysis thereby allowing the microorganic cells to be accumulated to a high density in the fermentation part.

In the case that the microorganic cells are not separated from the fermentation liquor before the fermentation liquor is introduced into the electrodialytic apparatus, however, it is necessary to use a device capable of preventing the microorganic cells from being deposited inside the electrodialytic apparatus and, at the same time, controlling the density of microorganic cells below a stated level. In the case of ordinary lactic acid bacteria, the density of the cells thereof is desired to be repressed below 50 g/liter on wet basis. For the control of the density of microorganic cells, a method which comprises discarding part of the cells being returned to the fermentation part at the outlet of the electrodialytic apparatus may be used, for example.

In the case that the microorganic cells are separated from the fermentation liquor by passage through a membrane or by means of centrifugation before the fermentation liquor is introduced into the electrodialytic apparatus, the density of microorganic cells in the fermentation liquor is not particularly limited but may be controlled by the capacity of the device for separation with a membrane or by the centrifugal separation device. In this case, a method which effects the control of the density of microorganic cells by discarding part of the microorganic cells at the outlet of the device serving to separate microorganic cells may be used, for example.

Now, this invention will be described specifically below with reference to working examples thereof.

### Example 1

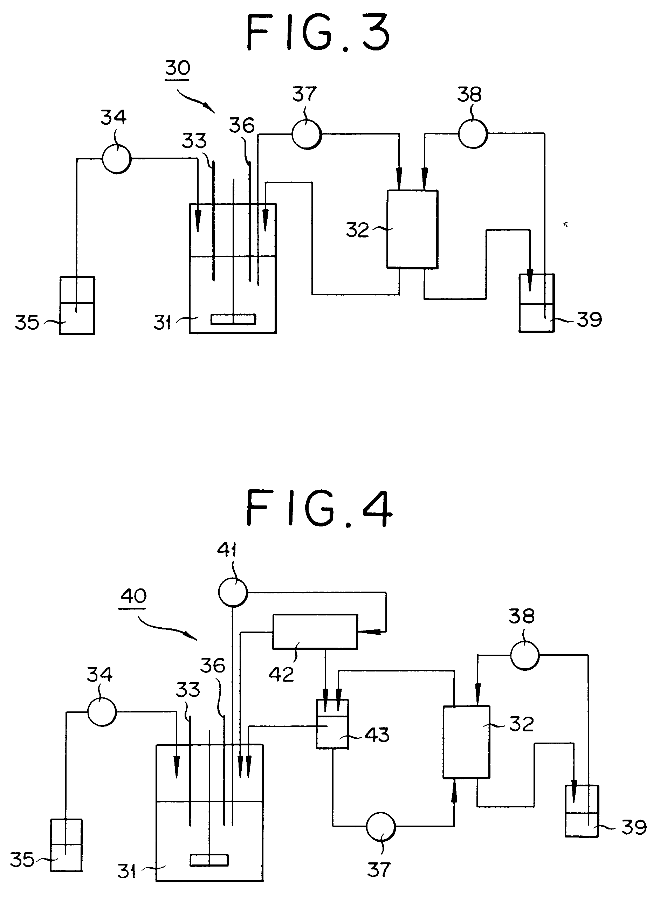

The method for fermentative production of lactic acid according to this invention was worked by the use of an experimental instrument constructed as illustrated in Fig. 3 for producing lactic acid by electrodialytic fermentation as remarked in this example.

As illustrated in Fig. 3, in an experimental instrument 30 for production of lactic acid by electrodialytic fermentation, a jar fermenter 31 having an inner volume of 3 liters and an electrodialytic apparatus 32 (produced by Asahi Chemical Industry Co., Ltd. and marketed under trademark designation of Microacylizer; available electrification surface area 0.55 dm², 15 cp, ion-exchange membrane cartridge AC-220) were interconnected to form a circulation system. To the jar fermenter 31, 1920 ml of a culture medium containing 200 g of glucose and 80 g of corn steep liquor and sterilized by means of an autoclave was supplied. To the culture medium in the jar fermenter 31, 80 ml of a seed culture liquor obtained preparatorily by culturing Lactobacillus rhamnosus IF0 3863 in a culture medium of the composition shown in Table 1 below was added to initiate fermentation.

**Table 1**

| Components of culture medium | Contents (g/liter of culture medium) |
|---|---|
| Yeast extract | 5.5 |
| Peptone | 12.5 |
| Glucose | 11.0 |
| KH₂PO₄ | 0.25 |
| K₂HPO₄ | 0.25 |
| Sodium acetate | 10.0 |
| MgSO₄ | 0.1 |
| MnSO₄ | 0.005 |
| FeSO₄ | 0.005 |

A heater and a stirrer attached to the jar fermenter 31 were actuated and operated at a fermentation temperature of 42°C and a stirring rate of 100 rpm and a pH electrode 33 attached also to the jar fermenter 31 was energized to measure the pH of the culture medium for fermentation in the jar fermenter. When the pH thus measured was below the prescribed level, the pH electrode 33 issued a signal to actuate an aqua ammonia feed pump 34 and feed a 10% aqua ammonia from an aqua ammonia storage vessel 35 to the jar fermenter 31 so as to maintain the pH of the culture medium for fermentation in the neighborhood of 6.0.

Then, after the start of the fermentation, when the electroconductivity of the culture medium for fermentation as measured by an electroconductivity electrode 36 attached to the jar fermenter 31 reached 26 mS/cm, a fermentation liquor circulation pump 37 was actuated to feed the fermentation liquor to the electrodialytic apparatus 32 and, at the same time, the electroconductivity electrode 36 issued a signal to start power supply to the electrodes of the electrodialytic apparatus 32. When the electroconductivity measured by the electroconductivity electrode 36 reached 12 mS/cm, the electroconductivity electrode 36 issued a signal to stop the power supply. The power supply to the electrodes was suspended until the electroconductivity measured by the electroconductivity electrode 36 returned to 26 mS/cm. A recovered aqueous ammonium lactate solution circulating pump 38 was actuated at the same time that the operation of the fermentation liquor circulating pump 37 was started so as to recover concentrated and isolated ammonium lactate in an aqueous ammonium lactate solution recovering vessel 39. Thereafter, the fermentation was continued by similarly performing ON-OFF control of the power supply to the electrodes of the electrodialytic apparatus 32. The electrification was implemented at a fixed magnitude of 1.0 A. The fermentation liquor circulating pump 37 and the aqueous ammonium lactate solution recovering vessel 39 were operated incessantly after the start of the operation of the system until the completion of the fermentation without reference to the presence/absence of the power supply to the electrodes of the electrodialytic apparatus 32.

Owing to the control of electrodialysis by dint of the electroconductivity mentioned above, the ammonium lactate concentration in the fermentation liquor was constantly kept in the range of from 2.0 to 1.0% by weight and the fermentation was completed in 36 hours. The total time of electrification was 4.0 hours. The leak of glucose on the recovering vessel 39 side was 5.9% by weight based on the weight of glucose initially charged.

When the same culture medium was subjected to the ordinary batchwise fermentation, the total time required for complete fermentation was 57 hours.

### Example 2

A fermentation was carried out by following the procedure of Example 1 excepting inorganic salts shown in Table 2 below were added to the culture medium for fermentation in the jar fermenter 32 at the end of each batch of electrodialysis by the ON-OFF control of electrodialysis.

**Table 2**

| Composition of inorganic salts | Amount added (g) |
|---|---|
| KH₂PO₄ | 0.8 |
| MgSO₄·7H₂O | 0.2 |
| NaCℓ | 0.04 |

The fermentation proceeded substantially as smoothly as in Example 1 and was completed in 36 hours. The total time of electrification was 4.3 hours. The leak of glucose on the recovering vessel 39 side was 6.7% by weight based on the weight of glucose initially charged.

### Example 3

A fermentation was carried out in the same manner as in Example 1 while continuing the ON-OFF control of the power supply to the electrodes of the electrodialytic apparatus 32 so that the electroconductivity measured by the electroconductivity electrode 36 would be kept in the range of from 6 to 13 mS/cm. The power supply was made at a fixed magnitude of 0.7 A.

The ammonium lactate concentration in the fermentation liquor was constantly maintained in the range of from 0.5 to 1.0% by weight and the time for completion of the fermentation was shortened to 28 hours. The total time of electrification was 6.8 hours. The leak of glucose on the recovering vessel 39 side was 9.6% by weight based on the weight of initially charged glucose.

### Example 4

The method for fermentative production of lactic acid according to this invention was worked by the use of an experimental instrument constructed as illustrated in Fig. 4 for producing lactic acid by electrodialytic fermentation as remarked in this example.

An experimental instrument 40 of Fig. 4 was equal to the experimental instrument of Fig. 3 indicated in Example 1, excepting a circulation pump 41 serving a precision filtration membrane module, a precision filtration membrane module 42 (produced by Asahi Medical K.K. and marketed under product code of "AHF-Ma," using cellulose diacetate hollow fibers 370µm in inside diameter, 183 mm in length, and 0.2µm in pore size), and a liquid reservoir 43 interconnecting the precision filtration membrane module 42 and the electrodialytic apparatus 32 are interposed between the fermentation part 31 and the electrodialytic apparatus 32. In the actual operation of this experimental instrument, the fermentation was carried out by following the procedure of Example 1 while after the electroconductivity measured by the electroconductivity electrode 36 had reached 26 mS/cm, actuating the circulation pump 41 for the precision filtration membrane module so as to forward the fermentation liquor to the precision filtration membrane module 42 and deliver the fermentation liquor remaining after the separation of microorganic cells by means of the membrane to the liquid reservoir 43 and further actuating the fermentation liquor circulating pump 37 so as to feed the fermentation liquor in the liquid reservoir 43 to the electrodialytic apparatus 32 to be electrodialyzed therein. The circulation pump 41 serving the precision filtration membrane module was operated incessantly after the start of the operation of the system until the completion of the fermentation without reference to the presence/absence of the power supply to the electrodes of the electrodialytic apparatus 32.

The ammonium lactate concentration in the fermentation liquor was maintained in the range of from 2.0 to 1.0% by weight and the time required for completion of the fermentation was 36 hours, the same duration as in Example 1. The total time of power supply was 3.5 hours. The leak of glucose on the recovering vessel 39 side was 5.9% by weight based on the weight of initially charged glucose.

## Claims

1. A method for the fermentative production of lactic acid characterised by circulating a fermentation liquor between a fermentation section (11) and an electrodialytic apparatus (12) culturing a microorganism arranged to accumulate a lactate in a culture medium in the fermentation section and removing the lactate from the fermentation liquor using the electrodialytic apparatus (12).

2. A method as claimed in Claim 1, characterised in that the operation of the electrodialytic apparatus (12) is carried out intermittently.

3. A method as claimed in Claim 1 or Claim 2, characterised in that fermentation liquors from the plurality of fermentation sections (11) are alternately circulated between the electrodialytic apparatus (12) and the fermentation sections (11).

4. A method as claimed in Claim 2 or Claim 3, characterised in that the raw materials for fermentation are supplied by way of replenishment to the fermentation sections (11) while operation of the electrodialytic apparatus is suspended.

5. A method as claimed in Claim 4, characterised in that the raw materials for fermentation supplied by way of partial replenishment to the fermentation sections are sugars and/or inorganic salts.

6. A method as claimed in any preceding Claim, characterised in that the operation of the electrodialytic apparatus (12) is controlled by the degree of electroconductivity of the culture medium for fermentation.

7. A method as claimed in any preceding Claim, characterised in that the microorganism capable of accumulating a lactate is a microorganism belonging to one of the genera Lactobacillus, Lactococcus, Bacillus, and Sporolactobacillus.

8. A method as claimed in any preceding Claim, characterised in that the cells of the microorganism are separated from the fermentation liquor through the medium of a microorganic cell separation device (42) prior to the fermentation liquor being led to the electrodialytic apparatus.

9. A method as claimed in any of Claims 1 to 6, characterised in that the microorganism arranged to accumulate the lactate is a microorganism belonging to gen Rhizopus and the fermentation liquor having microorganic cells therein is sterilised through the medium of a sterilising device prior to being led to the electrodialytic apparatus (12).

10. A method as claimed in any preceding Claim, characterised in that the culture medium and the microorganic cells in any fermentation section (11) are partly extracted and a fresh supply of culture medium is added to the respective fermentation section (11) to allow the fermentation to proceed continuously.

11. A method as claimed in any preceding Claim, characterised in that the lactate is ammonium lactate and the pH of the culture medium for fermentation is controlled by ammonia.
